# EUROPEAN PATENT SPECIFICATION

(11) **EP 1 297 005 B1**
(45) Date of publication and mention of the grant of the patent: **26.08.2009**
(21) Application number: 01954278.6
(22) Date of filing: 03.07.2001
(51) Int. Cl.: C07K 14/295, C12N 15/31, A61K 39/118

(54) **IMMUNISATION AGAINST CHLAMYDIA PNEUMONIAE**
IMMUNISIERUNG GEGEN CHLAMYDIA PNEUMONIAE
IMMUNISATION CONTRE UNE INFECTION PAR CHLAMYDIA PNEUMONIAE

(30) Priority: 03.07.2000 GB 0016363; 11.07.2000 GB 0017047; 21.07.2000 GB 0017983; 07.08.2000 GB 0019368; 18.08.2000 GB 0020440; 14.09.2000 GB 0022583; 10.11.2000 GB 0027549; 22.12.2000 GB 0031706
(43) Date of publication of application: 02.04.2003
(60) Divisional application: 09075245.2
(73) Proprietor: Novartis Vaccines and Diagnostics S.r.l., 53100 Siena (SI) (IT)
(72) Inventor: RATTI, Giulio, 53100 Siena (IT); GRANDI, Guido, 53100 Siena (IT)
(74) Representative: Marshall, Cameron John
(86) International application number: PCT/IB2001/001445
(87) International publication number: WO 2002/002606

(56) References cited:
- WO-A-00/34483
- WO-A-00/34483
- WO-A-01/75113
- WO-A-99/27105
- US-A- 5 869 608
- US-A- 5 869 608
- DATABASE UNIPROT Accession number Q9Z827 1 May 1999 (1999-05-01), KALMAN, S. ET AL.: "CT398 hypothetical protein" XP002292278 & KALMAN S. ET AL.: "Comparative genomes of Chlamydia pneumoniae and C. trachomatis" NATURE GENETICS, vol. 21, no. 4, 1999, pages 385-389,
- DATABASE EMBL [Online] Accession number AE001589 15 March 1999 (1999-03-15), KALMAN S. ET AL.: "Chlamydia pneumoniae section 5 of 103 of the complete genome" XP002185615 accession no. AE001589 & KALMAN S. ET AL.: "Comparative genomes of Chlamydia pneumoniae and C. trachomatis" NATURE GENETICS, vol. 21, no. 4, 1999, pages 385-389,
- DATABASE EMBL Accession number AE001637 15 March 1999 (1999-03-15), KALMAN S. ET AL.: "Chlamydia pneumoniae section 53 of 103 of the complete genome" XP002292279 & SUE KALMAN ET AL.: "Comparative genomes of Chlamydia pneumoniae and C. trachomatis" NATURE GENETICS, vol. 21, no. 4, 1999, pages 385-389,
- DATABASE EMBL Accession number AE002183 14 March 2000 (2000-03-14), READ, T.D. ET AL.: "Chlamydophila pneumoniae AR39, section 18 of 94 of the complete genome" XP002292280 & T.D. READ ET AL.: "Genome sequences of Chlamydia trachomatis MoPn and Chlamydia pneumoniae AR39" NUCLEIC ACIDS RESEARCH, vol. 28, no. 6, 2000, pages 1397-1406, XP002164566
- DATABASE EMBL [Online] Accession number AE001589, 15 March 1999 (1999-03-15) KALMAN S. ET AL.: "Chlamydia pneumoniae section 5 of 103 of the complete genome" XP002185615 & KALMAN S. ET AL.: "Comparative genomes of Chlamydia pneumoniae and C. trachomatis" NATURE GENETICS, vol. 21, no. 4, 1999, pages 385-389,
- GIULIO RATTI ET AL.: "Searching the Chlamydia genomes for new vaccine candidates" GENOMICS, PROTEOMICS AND VACCINES, 2004, pages 245-266,

## Description

### TECHNICAL FIELD

This invention is in the field of immunisation against chlamydial infection, in particular against infection by *Chlamydia pneumoniae.*

### BACKGROUND ART

*Chlamydiae* are obligate intracellular parasites of eukaryotic cells which are responsible for endemic sexually transmitted infections and various other disease syndromes. They occupy an exclusive eubacterial phylogenic branch, having no close relationship to any other known organisms - they are classified in their own order *(Chlamydiales)* which contains a single family *(Chlamydiaceae)* which in turn contains a single genus *(Chlamydia).* A particular characteristic of the *Chlamydiae* is their unique life cycle, in which the bacterium alternates between two morphologically distinct forms: an extracellular infective form (elementary bodies, EB) and an intracellular non-infective form (reticulate bodies, RB). The life cycle is completed with the re-organization of RB into EB, which subsequently leave the disrupted host cell ready to infect further cells.

Four chlamydial species are currently known - *C.trachomatis, C.pneumoniae, C.pecorum* and

*C.psittaci* [*e.g.* Raulston (1995) Mol Microbiol 15:607-616; Everett (2000) Vet Microbiol 75:109-126]. *C.pneumoniae* is closely related to *C.trachomatis,* as the whole genome comparison of at least two isolates from each species has shown [Kalman et al. (1999) Nature Genetics 21:385-389; Read et al. (2000) Nucleic Acids Res 28:1397-406; Stephens et al. (1998) Science 282:754-759]. Based on surface reaction with patient immune sera, the current view is that only one serotype of *C.pneumoniae* exists world-wide.

*C.pneumoniae* is a common cause of human respiratory disease. It was first isolated from the conjunctiva of a child in Taiwan in 1965, and was established as a major respiratory pathogen in 1983. In the USA, *C.pneumoniae* causes approximately 10% of community-acquired pneumonia and 5% of pharyngitis, bronchitis, and sinusitis.

More recently, the spectrum of *C.pneumoniae* infections has been extended to include atherosclerosis, coronary heart disease, carotid artery stenosis, myocardial infarction, cerebrovascular disease, aortic aneurysm, claudication, and stroke. The association of *C.pneumoniae* with atherosclerosis is corroborated by the presence of the organism in atherosclerotic lesions throughout the arterial tree and the near absence of the organism in healthy arterial tissue. *C.pneumoniae* has also been isolated from coronary and carotid atheromatous plaques. The bacterium has also been associated with other acute and chronic respiratory diseases (*e.g*. otitis media, chronic obstructive pulmonary disease, pulmonary exacerbation of cystic fibrosis) as a result of sero-epidemiologic observations, case reports, isolation or direct detection of the organism in specimens, and successful response to anti-chlamydial antibiotics. To determine whether chronic infection plays a role in initiation or progression of disease, intervention studies in humans have been initiated, and animal models of *C.pneumoniae* infection have been developed.

Considerable knowledge of the epidemiology of *C.pneumoniae* infection has been derived from serologic studies using the *C.pneumoniae-specific* microimmunofluorescence test. Infection is ubiquitous, and it is estimated that virtually everyone is infected at some point in life, with common re-infection. Antibodies against *C.pneumoniae* are rare in children under the age of 5, except in developing and tropical countries. Antibody prevalence increases rapidly at ages 5 to 14, reaching 50% at the age of 20, and continuing to increase slowly to ∼80% by age 70.

A current hypothesis is that *C.pneumoniae* can persist in an asymptomatic low-grade infection in very large sections of the human population. When this condition occurs, it believed that the presence of *C.pneumoniae,* and/or the effects of the host reaction to the bacterium, can cause or help progress of cardiovascular illness.

It is not yet clear whether *C.pneumoniae* is actually a causative agent of cardiovascular disease, or whether it is just artefactually associated with it. It has been shown, however, that *C.pneumoniae* infection can induce LDL oxidation by human monocytes [Kalayoglu et al. (1999) J. Infect. Dis. 180:780-90; Kalayoglu et al. (1999) Am. Heart J. 138:S488-490]. As LDL oxidation products are highly atherogenic, this observation provides a possible mechanism whereby *C.pneumoniae* may cause atheromatous degeneration. If a causative effect is confirmed, vaccination (prophylactic and therapeutic) will be universally recommended.

Genomic sequence information has been published for *C.pneumoniae* [Kalman *et al.* (1999) *supra;* Read *et al.* (2000) *supra;* Shirai et al. (2000) J. Infect. Dis. 181(Suppl 3):S524-S527; WO99/27105; WO00/27994] and is available from GenBank. Sequencing efforts have not, however, focused on vaccination, and the availability of genomic sequence does not in itself indicate which of the >1000 genes might encode useful antigens for immunisation and vaccination. WO99/27105, for instance, implies that every one of the 1296 ORFs identified in the *C.pneumoniae* strain CM1 genome is a useful vaccine antigen.

Database EMBL Accession number AE001637 15 March 1999, Kalman, S. et al., *"Chlamydia pneumoniae* section 53 of 103 of the complete genome" discloses a *C.pneumoniae* nucleotide sequence which encodes several polypeptides, amongst which is a hypothetical protein designated CT398 which is 100% identical to SEQ ID NO:117. The nucleotide sequence comprises a section 100% identical to SEQ ID NO: 118. Likewise, database UNIPROT Accession number Q9Z827 1 May 1999, Kalman, S. et al., "CT398 hypothetical protein" discloses a hypothetical *C. pneumoniae* protein CT398 which is 100% identical to SEQ ID NO: 117. Neither of the database entries suggests a medical use for these sequences.

It is an object of the present invention to identify antigens useful for vaccine production and development from amongst the many proteins present in *C.pneumoniae.* It is a further object to identify antigens useful for diagnosis (*e.g*. immunodiagnosis) of *C.pneumoniae.*

### DISCLOSURE OF THE INVENTION

The invention provides a protein comprising amino acid sequence SEQ ID NO: 117 for use in the treatment or prevention of infection due to Chlamydia bacteria. The protein may be used in the manufacture of a medicament for the treatment or prevention of infection due to Chlamydia bacteria.

The proteins of the invention can, of course, be prepared by various means (*e.g*. native expression, recombinant expression, purification from cell culture, chemical synthesis *etc.)* and in various forms (*e.g*. native, fusions *etc.).* They are preferably prepared in substantially pure form (*ie*. substantially free from other *C.pneumoniae* or host cell proteins). Heterologous expression in *E.coli* is a preferred preparative route.

A summary of standard techniques and procedures which may be employed in order to perform the invention (*e.g*. to utilise the disclosed sequences for immunisation) follows. This summary is not a limitation on the invention but, rather, gives examples that may be used, but are not required.

### General

The practice of the present invention will employ, unless otherwise indicated, conventional techniques of molecular biology, microbiology, recombinant DNA, and immunology, which are within the skill of the art. Such techniques are explained fully in the literature *e.g*. Sambrook Molecular Cloning; A Laboratory Manual, Second Edition (1989) and Third Edition (2001); DNA Cloning, Volumes I and ii (D.N Glover ed. 1985); Oligonucleotide Synthesis (M.J. Gait ed, 1984); Nucleic Acid Hybridization (B.D. Hames & S.J. Higgins eds. 1984); Transcription and Translation (B.D. Hames & S.J. Higgins eds. 1984); Animal Cell Culture (R.I. Freshney ed. 1986); Immobilized Cells and Enzymes (IRL Press, 1986); B. Perbal, A Practical Guide to Molecular Cloning (1984); the Methods in Enzymology series (Academic Press, Inc.), especially volumes 154 & 155; Gene Transfer Vectors for Mammalian Cells (J.H. Miller and M.P. Calos eds. 1987, Cold Spring Harbor Laboratory); Mayer and Walker, eds. (1987), Immunochemical Methods in Cell and Molecular Biology (Academic Press, London); Scopes, (1987) Protein Purification: Principles and Practice, Second Edition (Springer-Verlag, N.Y.), and Handbook of Experimental Immunology, Volumes I-IV (D.M. Weir and C. C. Blackwell eds 1986).

Standard abbreviations for nucleotides and amino acids are used in this specification.

### Definitions

A composition containing X is "substantially free of" Y when at least 85% by weight of the total X+Y in the composition is X. Preferably, X comprises at least about 90% by weight of the total of X+Y in the composition, more preferably at least about 95% or even 99% by weight.

The term "comprising" means "including" as well as "consisting" *e.g.* a composition "comprising" X may consist exclusively of X or may include something additional to X, such as X+Y.

### Expression systems

The Chlamydial nucleotide sequences can be expressed in a variety of different expression systems; for example those used with mammalian cells, baculoviruses, plants, bacteria, and yeast.

### i. Mammalian Systems

Mammalian expression systems are known in the art. A mammalian promoter is any DNA sequence capable of binding mammalian RNA polymerase and initiating the downstream (3') transcription of a coding sequence *(e.g.* structural gene) into mRNA. A promoter will have a transcription initiating region, which is usually placed proximal to the 5' end of the coding sequence, and a TATA box, usually located 25-30 base pairs (bp) upstream of the transcription initiation site. The TATA box is thought to direct RNA polymerase II to begin RNA synthesis at the correct site. A mammalian promoter will also contain an upstream promoter element, usually located within 100 to 200 bp upstream of the TATA box. An upstream promoter element determines the rate at which transcription is initiated and can act in either orientation [Sambrook et al. (1989) "Expression of Cloned Genes in Mammalian Cells." In Molecular Cloning: A Laboratory Manual, 2nd ed*.].*

Mammalian viral genes are often highly expressed and have a broad host range; therefore sequences encoding mammalian viral genes provide particularly useful promoter sequences. Examples include the SV40 early promoter, mouse mammary tumor virus LTR promoter, adenovirus major late promoter (Ad MLP), and herpes simplex virus promoter. In addition, sequences derived from non-viral genes, such as the murine metallotheionein gene, also provide useful promoter sequences. Expression may be either constitutive or regulated (inducible), depending on the promoter can be induced with glucocorticoid in hormone-responsive cells.

The presence of an enhancer element (enhancer), combined with the promoter elements described above, will usually increase expression levels. An enhancer is a regulatory DNA sequence that can stimulate transcription up to 1000-fold when linked to homologous or heterologous promoters, with synthesis beginning at the normal RNA start site. Enhancers are also active when they are placed upstream or downstream from the transcription initiation site, in either normal or flipped orientation, or at a distance of more than 1000 nucleotides from the promoter [Maniatis et al. (1987) Science 236:1237; Alberts et al. (1989) Molecular Biology of the Cell, 2nd ed.].

Enhancer elements derived from viruses may be particularly useful, because they usually have a broader host range. Examples include the SV40 early gene enhancer [Dijkema et al (1985) EMBO J. 4:761] and the enhancer/promoters derived from the long terminal repeat (LTR) of the Rous Sarcoma Virus [Gorman et al. (1982) PNAS USA 79:6777] and from human cytomegalovirus [Boshart et al. (1985) Cell 41:521]. Additionally, some enhancers are regulatable and become active only in the presence of an inducer, such as a hormone or metal ion [Sassone-Corsi and Borelli (1986) Trends Genet. 2:215; Maniatis et al. (1987) Science 236:1237].

A DNA molecule may be expressed intracellularly in mammalian cells. A promoter sequence may be directly linked with the DNA molecule, in which case the first amino acid at the N-terminus of the recombinant protein will always be a methionine, which is encoded by the ATG start codon. If desired, the N-terminus may be cleaved from the protein by *in vitro* incubation with cyanogen bromide.

Alternatively, foreign proteins can also be secreted from the cell into the growth media by creating chimeric DNA molecules that encode a fusion protein comprised of a leader sequence fragment that provides for secretion of the foreign protein in mammalian cells. Preferably, there are processing sites encoded between the leader fragment and the foreign gene that can be cleaved either *in vivo* or *in vitro.* The leader sequence fragment usually encodes a signal peptide comprised of hydrophobic amino acids which direct the secretion of the protein from the cell. The adenovirus triparite leader is an example of a leader sequence that provides for secretion of a foreign protein in mammalian cells.

Usually, transcription termination and polyadenylation sequences recognized by mammalian cells are regulatory regions located 3' to the translation stop codon and thus, together with the promoter elements, flank the coding sequence. The 3' terminus of the mature mRNA is formed by site-specific post-transcriptional cleavage and polyadenylation [Birnstiel et al. (1985) Cell 41:349; Proudfoot and Whitelaw (1988) "Termination and 3' end processing of eukaryotic RNA. In Transcription and splicing (ed. B.D. Hames and D.M. Glover); Proudfoot (1989) Trends Biochem. Sci. 14:105]. These sequences direct the transcription of an mRNA which can be translated into the polypeptide encoded by the DNA. Examples of transcription terminater/polyadenylation signals include those derived from SV40 [Sambrook et al (1989) "Expression of cloned genes in cultured mammalian cells." In Molecular Cloning: A Laboratory Manual].

Usually, the above described components, comprising a promoter, polyadenylation signal, and transcription termination sequence are put together into expression constructs. Enhancers, introns with functional splice donor and acceptor sites, and leader sequences may also be included in an expression construct, if desired. Expression constructs are often maintained in a replicon, such as an extrachromosomal element (*e.g*. plasmids) capable of stable maintenance in a host, such as mammalian cells or bacteria. Mammalian replication systems include those derived from animal viruses, which require trans-acting factors to replicate. For example, plasmids containing the replication systems of papovaviruses, such as SV40 [Gluzman (1981) Cell 23:175] or polyomavirus, replicate to extremely high copy number in the presence of the appropriate viral T antigen. Additional examples of mammalian replicons include those derived from bovine papillomavirus and Epstein-Barr virus. Additionally, the replicon may have two replicaton systems, thus allowing it to be maintained, for example, in mammalian cells for expression and in a prokaryotic host for cloning and amplification. Examples of such mammalian-bacteria shuttle vectors include pMT2 [Kaufman et al. (1989) Mol. Cell. Biol. 9:946] and pHEBO [Shimizu et al. (1986) Mol. Cell. Biol. 6:1074].

The transformation procedure used depends upon the host to be transformed. Methods for introduction of heterologous polynucleotides into mammalian cells are known in the art and include dextran-mediated transfection, calcium phosphate precipitation, polybrene-mediated transfection, protoplast fusion, electroporation, encapsulation of polynucleotide(s) in liposomes, direct microinjection of the DNA into nuclei.

Mammalian cell lines available as hosts for expression are known in the art and include many immortalized cell lines available from the American Type Culture Collection (ATCC), including but not limited to, Chinese hamster ovary (CHO) cells, HeLa cells, baby hamster kidney (BHK) cells, monkey kidney cells (COS), human hepatocellular carcinoma cells *(e.g.* Hep G2), and a number of other cell lines.

### ii. Baculovirus Systems

The polynucleotide encoding the protein can also be inserted into a suitable insect expression vector, and is operably linked to the control elements within that vector. Vector construction employs techniques which are known in the art. Generally, the components of the expression system include a transfer vector, usually a bacterial plasmid, which contains both a fragment of the baculovirus genome, and a convenient restriction site for insertion of the heterologous gene or genes to be expressed; a wild type baculovirus with a sequence homologous to the baculovirus-specific fragment in the transfer vector (this allows for the homologous recombination of the heterologous gene in to the baculovirus genome); and appropriate insect host cells and growth media.

After inserting the DNA sequence encoding the protein into the transfer vector, the vector and the wild type viral genome are transfected into an insect host cell where the vector and viral genome are allowed to recombine. The packaged recombinant virus is expressed and recombinant plaques are identified and purified. Materials and methods for baculovirus/insect cell expression systems are commercially available in kit form from, *inter alia,* Invitrogen, San Diego CA ("MaxBac" kit). These techniques are generally known to those skilled in the art and fully described in Summers and Smith, Texas Agricultural Experiment Station Bulletin No. 1555 (1987) (hereinafter "Summers and Smith").

Prior to inserting the DNA sequence encoding the protein into the baculovirus genome, the above described components, comprising a promoter, leader (if desired), coding sequence of interest, and transcription termination sequence, are usually assembled into an intermediate transplacement construct (transfer vector). This construct may contain a single gene and operably linked regulatory elements; multiple genes, each with its owned set of operably linked regulatory elements; or multiple genes, regulated by the same set of regulatory elements. Intermediate transplacement constructs are often maintained in a replicon, such as an extrachromosomal element (*e.g*. plasmids) capable of stable maintenance in a host, such as a bacterium. The replicon will have a replication system, thus allowing it to be maintained in a suitable host for cloning and amplification.

Currently, the most commonly used transfer vector for introducing foreign genes into AcNPV is pAc373. Many other vectors, known to those of skill in the art, have also been designed. These include, for example, pVL985 (which alters the polyhedrin start codon from ATG to ATT, and which introduces a BamHI cloning site 32 basepairs downstream from the ATT; see Luckow and Summers, Virology (1989) 17:31.

The plasmid usually also contains the polyhedrin polyadenylation signal (Miller et al. (1988) Ann. Rev. Microbiol., 42:177) and a prokaryotic ampicillin-resistance *(amp)* gene and origin of replication for selection and propagation in *E. coli.*

Baculovirus transfer vectors usually contain a baculovirus promoter. A baculovirus promoter is any DNA sequence capable of binding a baculovirus RNA polymerase and initiating the downstream (5' to 3') transcription of a coding sequence (*e.g*. structural gene) into mRNA. A promoter will have a transcription initiation region which is usually placed proximal to the 5' end of the coding sequence. This transcription initiation region usually includes an RNA polymerase binding site and a transcription initiation site. A baculovirus transfer vector may also have a second domain called an enhancer, which, if present, is usually distal to the structural gene. Expression may be either regulated or constitutive.

Structural genes, abundantly transcribed at late times in a viral infection cycle, provide particularly useful promoter sequences. Examples include sequences derived from the gene encoding the viral polyhedron protein, Friesen et al., (1986) "The Regulation of Baculovirus Gene Expression," in: The Molecular Biology of Baculoviruses (ed. W alter Doerfler); EPO Publ. Nos. 127 839 and 155 476; and the gene encoding the p10 protein, Vlak et al., (1988), J. Gen. Virol. 69:765.

DNA encoding suitable signal sequences can be derived from genes for secreted insect or baculovirus proteins, such as the baculovirus polyhedrin gene (Carbonell et al. (1988) Gene, 73:409). Alternatively, since the signals for mammalian cell posttranslational modifications (such as signal peptide cleavage, proteolytic cleavage, and phosphorylation) appear to be recognized by insect cells, and the signals required for secretion and nuclear accumulation also appear to be conserved between the invertebrate cells and vertebrate cells, leaders of non-insect origin, such as those derived from genes encoding human α-interferon, Maeda et al., (1985), Nature 315:592; human gastrin-releasing peptide, Lebacq-Verheyden et al., (1988), Molec. Cell. Biol. 8:3129; human IL-2, Smith et al., (1985) Proc. Nat'l Acad. Sci. USA, 82:8404; mouse IL-3, (Miyajima et al., (1987) Gene 58:273; and human glucocerebrosidase, Martin et al. (1988) DNA, 7:99, can also be used to provide for secretion in insects.

A recombinant polypeptide or polyprotein may be expressed intracellularly or, if it is expressed with the proper regulatory sequences, it can be secreted. Good intracellular expression of nonfused foreign proteins usually requires heterologous genes that ideally have a short leader sequence containing suitable translation initiation signals preceding an ATG start signal. If desired, methionine at the N-terminus may be cleaved from the mature protein by *in vitro* incubation with cyanogen bromide.

Alternatively, recombinant polyproteins or proteins which are not naturally secreted can be secreted from the insect cell by creating chimeric DNA molecules that encode a fusion protein comprised of a leader sequence fragment that provides for secretion of the foreign protein in insects. The leader sequence fragment usually encodes a signal peptide comprised of hydrophobic amino acids which direct the translocation of the protein into the endoplasmic reticulum.

After insertion of the DNA sequence and/or the gene encoding the expression product precursor of the protein, an insect cell host is co-transformed with the heterologous DNA of the transfer vector and the genomic DNA of wild type baculovirus -- usually by co-transfection. The promoter and transcription termination sequence of the construct will usually comprise a 2-5kb section of the baculovirus genome. Methods for introducing heterologous DNA into the desired site in the baculovirus virus are known in the art. (See Summers and Smith *supra;* Ju et al. (1987); Smith et al., Mol. Cell. Biol. (1983) 3:2156; and Luckow and Summers (1989)). For example, the insertion can be into a gene such as the polyhedrin gene, by homologous double crossover recombination; insertion can also be into a restriction enzyme site engineered into the desired baculovirus gene. Miller et al., (1989), Bioessays 4:91. The DNA sequence, when cloned in place of the polyhedrin gene in the expression vector, is flanked both 5' and 3' by polyhedrin-specific sequences and is positioned downstream of the polyhedrin promoter.

The newly formed baculovirus expression vector is subsequently packaged into an infectious recombinant baculovirus. Homologous recombination occurs at low frequency (between ∼1% and ∼5%); thus, the majority of the virus produced after cotransfection is still wild-type virus. Therefore, a method is necessary to identify recombinant viruses. An advantage of the expression system is a visual screen allowing recombinant viruses to be distinguished. The polyhedrin protein, which is produced by the native virus, is produced at very high levels in the nuclei of infected cells at late times after viral infection. Accumulated polyhedrin protein forms occlusion bodies that also contain embedded particles. These occlusion bodies, up to 15µm in size, are highly refractile, giving them a bright shiny appearance that is readily visualized under the light microscope. Cells infected with recombinant viruses lack occlusion bodies. To distinguish recombinant virus from wild-type virus, the transfection supernatant is plaqued onto a monolayer of insect cells by techniques known to those skilled in the art. Namely, the plaques are screened under the light microscope for the presence (indicative of wild-type virus) or absence (indicative of recombinant virus) of occlusion bodies. "Current Protocols in Microbiology" Vol. 2 (Ausubel et al. eds) at 16.8 (Supp. 10, 1990); Summers & Smith, *supra;* Miller et al. (1989).

Recombinant baculovirus expression vectors have been developed for infection into several insect cells. For example, recombinant baculoviruses have been developed for, *inter alia: Aedes aegypti, Autographa californica, Bombyx mori, Drosophila melanogaster, Spodoptera frugiperda,* and *Trichoplusia ni* (WO 89/046699; Carbonell et al., (1985) J. Virol. 56:153; Wright (1986) Nature 321:718; Smith et al., (1983) Mol. Cell. Biol. 3:2156; and see generally, Fraser, et al. (1989) In Vitro Cell. Dev. Biol. 25:225).

Cells and cell culture media are commercially available for both direct and fusion expression of heterologous polypeptides in a baculovirus/expression system; cell culture technology is generally known to those skilled in the art. *See, e.g.* Summers and Smith *supra.*

The modified insect cells may then be grown in an appropriate nutrient medium, which allows for stable maintenance of the plasmid(s) present in the modified insect host. Where the expression product gene is under inducible control, the host may be grown to high density, and expression induced. Alternatively, where expression is constitutive, the product will be continuously expressed into the medium and the nutrient medium must be continuously circulated, while removing the product of interest and augmenting depleted nutrients. The product may be purified by such techniques as chromatography, e.g. HPLC, affinity chromatography, ion exchange chromatography, etc.; electrophoresis; density gradient centrifugation; solvent extraction, or the like. As appropriate, the product may be further purified, as required, so as to remove substantially any insect proteins which are also secreted in the medium or result from lysis of insect cells, so as to provide a product which is at least substantially free of host debris, e.g. proteins, lipids and polysaccharides.

In order to obtain protein expression, recombinant host cells derived from the transformants are incubated under conditions which allow expression of the recombinant protein encoding sequence. These conditions will vary, dependent upon the host cell selected. However, the conditions are readily ascertainable to those of ordinary skill in the art, based upon what is known in the art.

### iii. Plant Systems

There are many plant cell culture and whole plant genetic expression systems known in the art. Exemplary plant cellular genetic expression systems include those described in patents, such as: US 5,693,506; US 5,659,122; and US 5,608,143. Additional examples of genetic expression in plant cell culture has been described by Zenk, Phytochemistry 30:3861-3863 (1991). Descriptions of plant protein signal peptides may be found in addition to the references described above in Vaulcombe et al., Mol. Gen. Genet. 209:33-40 (1987); Chandler et al., Plant Molecular Biology 3:407-418 (1984); Rogers, J. Biol. Chem. 260:3731-3738 (1985); Rothstein et al., Gene 55:353-356 (1987); Whittier et al., Nucleic Acids Research 15:2515-2535 (1987); Wirsel et al., Molecular Microbiology 3:3-14 (1989); Yu et al., Gene 122:247-253 (1992). A description of the regulation of plant gene expression by the phytohormone, gibberellic acid and secreted enzymes induced by gibberellic acid can be found in R.L. Jones and J. MacMillin, Gibberellins: in: Advanced Plant Physiology,. Malcolm B. Wilkins, ed., 1984 Pitman Publishing Limited, London, pp. 21-52. References that describe other metabolically-regulated genes: Sheen, Plant Cell, 2:1027-1038(1990); Maas et al., EMBO J. 9:3447-3452 (1990); Benkel and Hickey, Proc. Natl. Acad. Sci. 84:1337-1339 (1987)

Typically, using techniques known in the art, a desired polynucleotide sequence is inserted into an expression cassette comprising genetic regulatory elements designed for operation in plants. The expression cassette is inserted into a desired expression vector with companion sequences upstream and downstream from the expression cassette suitable for expression in a plant host. The companion sequences will be of plasmid or viral origin and provide necessary characteristics to the vector to permit the vectors to move DNA from an original cloning host, such as bacteria, to the desired plant host. The basic bacterial/plant vector construct will preferably provide a broad host range prokaryote replication origin; a prokaryote selectable marker; and, for Agrobacterium transformations, T DNA sequences for Agrobacterium-mediated transfer to plant chromosomes. Where the heterologous gene is not readily amenable to detection, the construct will preferably also have a selectable marker gene suitable for determining if a plant cell has been transformed. A general review of suitable markers, for example for the members of the grass family, is found in Wilmink and Dons, 1993, Plant Mol. Biol. Reptr, 11 (2):165-185.

Sequences suitable for permitting integration of the heterologous sequence into the plant genome are also recommended. These might include transposon sequences and the like for homologous recombination as well as Ti sequences which permit random insertion of a heterologous expression cassette into a plant genome. Suitable prokaryote selectable markers include resistance toward antibiotics such as ampicillin or tetracycline. Other DNA sequences encoding additional functions may also be present in the vector, as is known in the art.

The nucleic acid molecules of the subject invention may be included into an expression cassette for expression of the protein(s) of interest. Usually, there will be only one expression cassette, although two or more are feasible. The recombinant expression cassette will contain in addition to the heterologous protein encoding sequence the following elements, a promoter region, plant 5' untranslated sequences, initiation codon depending upon whether or not the structural gene comes equipped with one, and a transcription and translation termination sequence. Unique restriction enzyme sites at the 5' and 3' ends of the cassette allow for easy insertion into a pre-existing vector.

A heterologous coding sequence may be for any protein relating to the present invention. The sequence encoding the protein of interest will encode a signal peptide which allows processing and translocation of the protein, as appropriate, and will usually lack any sequence which might result in the binding of the desired protein of the invention to a membrane. Since, for the most part, the transcriptional initiation region will be for a gene which is expressed and translocated during germination, by employing the signal peptide which provides for translocation, one may also provide for translocation of the protein of interest. In this way, the protein(s) of interest will be translocated from the cells in which they are expressed and may be efficiently harvested. Typically secretion in seeds are across the aleurone or scutellar epithelium layer into the endosperm of the seed. While it is not required that the protein be secreted from the cells in which the protein is produced, this facilitates the isolation and purification of the recombinant protein.

Since the ultimate expression of the desired gene product will be in a eucaryotic cell it is desirable to determine whether any portion of the cloned gene contains sequences which will be processed out as introns by the host's splicosome machinery. If so, site-directed mutagenesis of the "intron" region may be conducted to prevent losing a portion of the genetic message as a false intron code, Reed and Maniatis, Cell 41:95-105, 1985.

The vector can be microinjected directly into plant cells by use of micropipettes to mechanically transfer the recombinant DNA. Crossway, Mol. Gen. Genet, 202:179-185, 1985. The genetic material may also be transferred into the plant cell by using polyethylene glycol, Krens, et al., Nature, 296, 72-74, 1982. Another method of introduction of nucleic acid segments is high velocity ballistic penetration by small particles with the nucleic acid either within the matrix of small beads or particles, or on the surface, Klein, et al., Nature, 327, 70-73, 1987 and Knudsen and Muller, 1991, Planta, 185:330-336 teaching particle bombardment of barley endosperm to create transgenic barley. Yet another method of introduction would be fusion of protoplasts with other entities, either minicells, cells, lysosomes or other fusible lipid-surfaced bodies, Fraley, et al., Proc. Natl. Acad. Sci. USA, 79, 1859-1863, 1982.

The vector may also be introduced into the plant cells by electroporation. (Fromm et al., Proc. Natl Acad. Sci. USA 82:5824, 1985). In this technique, plant protoplasts are electroporated in the presence of plasmids containing the gene construct. Electrical impulses of high field strength reversibly permeabilize biomembranes allowing the introduction of the plasmids. Electroporated plant protoplasts reform the cell wall, divide, and form plant callus.

All plants from which protoplasts can be isolated and cultured to give whole regenerated plants can be transformed by the present invention so that whole plants are recovered which contain the transferred gene. It is known that practically all plants can be regenerated from cultured cells or tissues, including but not limited to all major species of sugarcane, sugar beet, cotton, fruit and other trees, legumes and vegetables. Some suitable plants include, for example, species from the genera *Fragaria, Lotus, Medicago, Onobrychis, Trifolium, Trigonella, Vigna, Citrus, Linum, Geranium, Manillot, Daucus, Arabidopsis, Brassica, Raphanus, Sinapis, Atropa, Capsicum, Datura, Hyoscyamus, Lycopersion, Nicotiana, Solanum, Petunia, Digitalis, Majorana, Cichorium, Helianthus, Lactuca, Bromus, Asparagus, Antirrhinum, Hererocallis, Nemesia, Pelargonium, Panicum, Pennisetum, Ranunculus, Senecio, Salpiglossis, Cucumis, Browaalia, Glycine, Lolium, Zea, Triticum, Sorghum,* and *Datura.*

Means for regeneration vary from species to species of plants, but generally a suspension of transformed protoplasts containing copies of the heterologous gene is first provided. Callus tissue is formed and shoots may be induced from callus and subsequently rooted. Alternatively, embryo formation can be induced from the protoplast suspension. These embryos germinate as natural embryos to form plants. The culture media will generally contain various amino acids and hormones, such as auxin and cytokinins. It is also advantageous to add glutamic acid and proline to the medium, especially for such species as corn and alfalfa. Shoots and roots normally develop simultaneously. Efficient regeneration will depend on the medium, on the genotype, and on the history of the culture. If these three variables are controlled, then regeneration is fully reproducible and repeatable.

In some plant cell culture systems, the desired protein of the invention may be excreted or alternatively, the protein may be extracted from the whole plant. Where the desired protein of the invention is secreted into the medium, it may be collected. Alternatively, the embryos and embryoless-half seeds or other plant tissue may be mechanically disrupted to release any secreted protein between cells and tissues. The mixture may be suspended in a buffer solution to retrieve soluble proteins. Conventional protein isolation and purification methods will be then used to purify the recombinant protein. Parameters of time, temperature pH, oxygen, and volumes will be adjusted through routine methods to optimize expression and recovery of heterologous protein.

### iv. Bacterial Systems

Bacterial expression techniques are known in the art. A bacterial promoter is any DNA sequence capable of binding bacterial RNA polymerase and initiating the downstream (3') transcription of a coding sequence (*e.g*. structural gene) into mRNA. A promoter will have a transcription initiation region which is usually placed proximal to the 5' end of the coding sequence. This transcription initiation region usually includes an RNA polymerase binding site and a transcription initiation site. A bacterial promoter may also have a second domain called an operator, that may overlap an adjacent RNA polymerase binding site at which RNA synthesis begins. The operator permits negative regulated (inducible) transcription, as a gene repressor protein may bind the operator and thereby inhibit transcription of a specific gene. Constitutive expression may occur in the absence of negative regulatory elements, such as the operator. In addition, positive regulation may be achieved by a gene activator protein binding sequence, which, if present is usually proximal (5') to the RNA polymerase binding sequence. An example of a gene activator protein is the catabolite activator protein (CAP), which helps initiate transcription of the lac operon in Escherichia coli (E. coli) [Raibaud et al. (1984) Annu. Rev. Genet. 18:173]. Regulated expression may therefore be either positive or negative, thereby either enhancing or reducing transcription.

Sequences encoding metabolic pathway enzymes provide particularly useful promoter sequences. Examples include promoter sequences derived from sugar metabolizing enzymes, such as galactose, lactose *(lac) [*Chang et al. (1977) Nature 198:1056], and maltose. Additional examples include promoter sequences derived from biosynthetic enzymes such as tryptophan (*trp*) [Goeddel et al. (1980) Nuc. Acids Res. 8:4057; Yelverton et al. (1981) Nucl. Acids Res. 9:731; US patent 4,738,921; EP-A-0036776 and EP-A-0121775]. The g-laotamase *(bla)* promoter system [Weissmann (1981) "The cloning of interferon and other mistakes." In Interferon 3 (ed. I. Gresser)], bacteriophage lambda PL [Shimatake et al. (1981) Nature 292:128] and T5 [US patent 4,689,406] promoter systems also provide useful promoter sequences.

In addition, synthetic promoters which do not occur in nature also function as bacterial promoters. For example, transcription activation sequences of one bacterial or bacteriophage promoter may be joined with the operon sequences of another bacterial or bacteriophage promoter, creating a synthetic hybrid promoter [US patent 4,551,433]. For example, the *tac* promoter is a hybrid *trp-lac* promoter comprised of both *trp* promoter and *lac* operon sequences that is regulated by the *lac* repressor [Amann et al. (1983) Gene 25:167; de Boer et al. (1983) Proc. Natl. Acad. Sci. 80:21]. Furthermore, a bacterial promoter can include naturally occurring promoters of non-bacterial origin that have the ability to bind bacterial RNA polymerase and initiate transcription. A naturally occurring promoter of non-bacterial origin can also be coupled with a compatible RNA polymerase to produce high levels of expression of some genes in prokaryotes. The bacteriophage T7 RNA polymerase/promoter system is an example of a coupled promoter system (Studier et al. (1986) J. Mol. Biol. 189:113; Tabor et al. (1985) Proc Natl. Acad. Sci. 82:1074]. In addition, a hybrid promoter can also be comprised of a bacteriophage promoter and an E. coli operator region (EPO-A-0 267 851).

In addition to a functioning promoter sequence, an efficient ribosome binding site is also useful for the expression of foreign genes in prokaryotes. In *E. coli,* the ribosome binding site is called the Shine-Dalgarno (SD) sequence and includes an initiation codon (ATG) and a sequence 3-9 nucleotides in length located 3-11 nucleotides upstream of the initiation codon [Shine et al. (1975) Nature 254:34]. The SD sequence is thought to promote binding of mRNA to the ribosome by the pairing of bases between the SD sequence and the 3' and of E. *coli* 16S rRNA [Steitz et al. (1979) "Genetic signals and nucleotide sequences in messenger RNA." In Biological Regulation and Development: Gene Expression (ed, R.F. Goldberger)]. To express eukaryotic genes and prokaryotic genes with weak ribosome-binding site [Sambrook et al. (1989) "Expression of cloned genes in Escherichia coli." In Molecular Cloning: A Laboratory Manual*].*

A DNA molecule may be expressed intracellularly. A promoter sequence may be directly linked with the DNA molecule, in which case the first amino acid at the N-terminus will always be a methionine, which is encoded by the ATG start codon. If desired, methionine at the N-terminus may be cleaved from the protein by *in vitro* incubation with cyanogen bromide or by either *in vivo* on *in vitro* incubation with a bacterial methionine N-terminal peptidase (EPO-A-0 219 237).

Fusion proteins provide an alternative to direct expression. Usually, a DNA sequence encoding the N-terminal portion of an endogenous bacterial protein, or other stable protein, is fused to the 5' end of heterologous coding sequences. Upon expression, this construct will provide a fusion of the two amino acid sequences. For example, the bacteriophage lambda cell gene can be linked at the 5' terminus of a foreign gene and expressed in bacteria.

The resulting fusion protein preferably retains a site for a processing enzyme (factor Xa) to cleave the bacteriophage protein from the foreign gene [Nagai et al. (1984) Nature 309:810]. Fusion proteins can also be made with sequences from the *lac*Z [Jia et al. (1987) Gene 60:197], *trpE* [Allen et al. (1987) J. Biotechnol. 5:93; Makoff et al. (1989) J. Gen. Microbiol. 135:11], and Chey [EP-A-0 324 647] genes. The DNA sequence at the junction of the two amino acid sequences may or may not encode a cleavable site. Another example is a ubiquitin fusion protein. Such a fusion protein is made with the ubiquitin region that preferably retains a site for a processing enzyme (*e.g*. ubiquitin specific processing-protease) to cleave the ubiquitin from the foreign protein. Through this method, native foreign protein can be isolated [Miller et al. (1989) Bio/Technology 7:698].

Alternatively, foreign proteins can also be secreted from the cell by creating chimeric DNA molecules that encode a fusion protein comprised of a signal peptide sequence fragment that provides for secretion of the foreign protein in bacteria [US patent 4,336,336]. The signal sequence fragment usually encodes a signal peptide comprised of hydrophobic amino acids which direct the secretion of the protein from the cell. The protein is either secreted into the growth media (gram-positive bacteria) or into the periplasmic space, located between the inner and outer membrane of the cell (gram-negative bacteria). Preferably there are processing sites, which can be cleaved either *in vivo* or *in vitro* encoded between the signal peptide fragment and the foreign gene.

DNA encoding suitable signal sequences can be derived from genes for secreted bacterial proteins, such as the *E. coli* outer membrane protein gene (*ompA*) [Masui et al. (1983), in: Experimental Manipulation of Gene Expression*;* Ghrayeb et al. (1984) EMBO J. 3:2437] and the *E. coli* alkaline phosphatase signal sequence (*phoA*) [Oka et al. (1985) Proc. Natl. Acad. Sci. 82:7212]. As an additional example, the signal sequence of the alpha-amylase gene from various Bacillus strains can be used to secrete heterologous proteins from *B. subtilis* [Palva et al. (1982) Proc. Natl. Acad. Sci. USA 79:5582; EP-A-0 244 042].

Usually, transcription termination sequences recognized by bacteria are regulatory regions located 3' to the translation stop codon, and thus together with the promoter flank the coding sequence. These sequences direct the transcription of an mRNA which can be translated into the polypeptide encoded by the DNA. Transcription termination sequences frequently include DNA sequences of about 50 nucleotides capable of forming stem loop structures that aid in terminating transcription. Examples include transcription termination sequences derived from genes with strong promoters, such as the *trp* gene in *E. coli* as well as other biosynthetic genes.

Usually, the above described components, comprising a promoter, signal sequence (if desired), coding sequence of interest, and transcription termination sequence, are put together into expression constructs. Expression constructs are often maintained in a replicon, such as an extrachromosomal element (*e.g*. plasmids) capable of stable maintenance in a host, such as bacteria. The replicon will have a replication system, thus allowing it to be maintained in a prokaryotic host either for expression or for cloning and amplification. In addition, a replicon may be either a high or low copy number plasmid. A high copy number plasmid will generally have a copy number ranging from about 5 to about 200, and usually about 10 to about 150. A host containing a high copy number plasmid will preferably contain at least about 10, and more preferably at least about 20 plasmids. Either a high or low copy number vector may be selected, depending upon the effect of the vector and the foreign protein on the host.

Alternatively, the expression constructs can be integrated into the bacterial genome with an integrating vector. Integrating vectors usually contain at least one sequence homologous to the bacterial chromosome that allows the vector to integrate. Integrations appear to result from recombinations between homologous DNA in the vector and the bacterial chromosome. For example, integrating vectors constructed with DNA from various Bacillus strains integrate into the Bacillus chromosome (EP-A- 0 127 328). Integrating vectors may also be comprised of bacteriophage or transposon sequences.

Usually, extrachromosomal and integrating expression constructs may contain selectable markers to allow for the selection of bacterial strains that have been transformed. Selectable markers can be expressed in the bacterial host and may include genes which render bacteria resistant to drugs such as ampicillin, chloramphenicol, erythromycin, kanamycin (neomycin), and tetracycline [Davies et al. (1978) Annu. Rev. Microbiol. 32:469]. Selectable markers may also include biosynthetic genes, such as those in the histidine, tryptophan, and leucine biosynthetic pathways.

Alternatively, some of the above described components can be put together in transformation vectors. Transformation vectors are usually comprised of a selectable market that is either maintained in a replicon or developed into an integrating vector, as described above.

Expression and transformation vectors, either extra-chromosomal replicons or integrating vectors, have been developed for transformation into many bacteria. For example, expression vectors have been developed for, *inter alia,* the following bacteria: Bacillus subtilis [Palva et al. (1982) Proc. Natl. Acad. Sci. USA 79:5582; EP-A-0 036 259 and EP-A-0 063 953; WO 84/04541], Escherichia coli [Shimatake et al. (1981) Nature 292:128; Amann et al. (1985) Gene 40:183; Studier et al. (1986) J. Mol. Biol. 189:113; EP-A-0 036 776,EP-A-0 136 829 and EP-A-0 136 907], Streptococcus cremoris [Powell et al. (1988) Appl. Environ. Microbiol. 54:655]; Streptococcus lividans [Powell et al. (1988) Appl. Environ. Microbiol. 54:655], Streptomyces lividans [US patent 4,745,056].

Methods of introducing exogenous DNA into bacterial hosts are well-known in the art, and usually include either the transformation of bacteria treated with CaCl₂ or other agents, such as divalent cations and DMSO. DNA can also be introduced into bacterial cells by electroporation. Transformation procedures usually vary with the bacterial species to be transformed. See *e.g*. [Masson et al. (1989) FEMS Microbiol. Lett. 60:273*;* Palva et al. (1982) Proc. Natl. Acad. Sci. USA 79:5582; EP-A-0 036 259 and EP-A-0 063 953; WO 84/04541, Bacillus], [Miller et al. (1988) Proc. Natl. Acad. Sci. 85:856; Wang et al. (1990) J. Bacteriol. 172:949, Campylobacter], [Cohen et al. (1973) Proc. Natl. Acad. Sci. 69:2110; Dower et al. (1988) Nucleic Acids Res. 16:6127; Kushner (1978) "An improved method for transformation of Escherichia coli with ColEl-derived plasmids. In Genetic Engineering: Proceedings of the International Symposium on Genetic Engineering (eds. H.W. Boyer and S. Nicosia); Mandel et al. (1970) J. Mol. Biol. 53:159; Taketo (1988) Biochim. Biophys. Acta 949:318; Escherichia], [Chassy et al. (1987) FEMS Microbiol. Lett. 44:173 Lactobacillus]; [Fiedler et al. (1988) Anal. Biochem 170:38, Pseudomonas]; [Augustin et al. (1990) FEMS Microbiol. Lett. 66:203, Staphylococcus], [Barany et al. (1980) J. Bacteriol. 144:698; Harlander (1987) "Transformation of Streptococcus lactis by electroporation, in: Streptococcal Genetics (ed. J. Ferretti and R. Curtiss III); Perry et al. (1981) Infect. Immun. 32:1295; Powell et al. (1988) Appl. Environ. Microbiol. 54:655; Somkuti et al. (1987) Proc. 4th Evr. Cong. Biotechnology 1:412, Streptococcus].

### v. Yeast Expression

Yeast expression systems are also known to one of ordinary skill in the art. A yeast promoter is any DNA sequence capable of binding yeast RNA polymerase and initiating the downstream (3') transcription of a coding sequence (*e.g*. structural gene) into mRNA. A promoter will have a transcription initiation region which is usually placed proximal to the 5' end of the coding sequence. This transcription initiation region usually includes an RNA polymerase binding site (the "TATA Box") and a transcription initiation site. A yeast promoter may also have a second domain called an upstream activator sequence (UAS), which, if present, is usually distal to the structural gene. The UAS permits regulated (inducible) expression. Constitutive expression occurs in the absence of a UAS. Regulated expression may be either positive or negative, thereby either enhancing or reducing transcription.

Yeast is a fermenting organism with an active metabolic pathway, therefore sequences encoding enzymes in the metabolic pathway provide particularly useful promoter sequences. Examples include alcohol dehydrogenase (ADH) (EP-A-0 284 044), enolase, glucokinase, glucose-6-phosphate isomerase, glyceraldehyde-3-phosphate-dehydrogenase (GAP or GAPDH), hexokinase, phosphofructokinase, 3-phosphoglycerate mutase, and pyruvate kinase (PyK) (EPO-A-0 329 203). The yeast *PH05* gene, encoding acid phosphatase, also provides useful promoter sequences [Myanohara et al. (1983) Proc. Natl. Acad. Sci. USA 80:1].

In addition, synthetic promoters which do not occur in nature also function as yeast promoters. For example, UAS sequences of one yeast promoter may be joined with the transcription activation region of another yeast promoter, creating a synthetic hybrid promoter. Examples of such hybrid promoters include the ADH regulatory sequence linked to the GAP transcription activation region (US Patent Nos. 4,876,197 and 4,880,734). Other examples of hybrid promoters include promoters which consist of the regulatory sequences of either the *ADH2, GAL4, GAL10,* OR *PH05* genes, combined with the transcriptional activation region of a glycolytic enzyme gene such as GAP or PyK (EP-A-0 164 556). Furthermore, a yeast promoter can include naturally occurring promoters of non-yeast origin that have the ability to bind yeast RNA polymerase and initiate transcription. Examples of such promoters include, *inter asia,* [Cohen et al. (1980) Proc. Natl. Acad. Sci. USA 77:1078; Henikoff et al. (1981) Nature 283:835; Hollenberg et al. (1981) Curr. Topics Microbiol. Immunol. 96:119; Hollenberg et al. (1979) "The Expression of Bacterial Antibiotic Resistance Genes in the Yeast Saccharomyces cerevisiae," in: Plasmids of Medical, Environmental and Commercial Importance (eds. K.N. Timmis and A. Puhler); Mercerau-Puigalon et al. (1980) Gene 11:163; Panthier et al. (1980) Curr. Genet. 2:109;].

A DNA molecule may be expressed intracellularly in yeast. A promoter sequence may be directly linked with the DNA molecule, in which case the first amino acid at the N-terminus of the recombinant protein will always be a methionine, which is encoded by the ATG start codon. If desired, methionine at the N-terminus may be cleaved from the protein by *in vitro* incubation with cyanogen bromide.

Fusion proteins provide an alternative for yeast expression systems, as well as in mammalian, baculovirus, and bacterial expression systems. Usually, a DNA sequence encoding the N-terminal portion of an endogenous yeast protein, or other stable protein, is fused to the 5' end of heterologous coding sequences. Upon expression, this construct will provide a fusion of the two amino acid sequences. For example, the yeast or human superoxide dismutase (SOD) gene, can be linked at the 5' terminus of a foreign gene and expressed in yeast. The DNA sequence at the junction of the two amino acid sequences may or may not encode a cleavable site. See *e.g*. EP-A-0 196 056. Another example is a ubiquitin fusion protein. Such a fusion protein is made with the ubiquitin region that preferably retains a site for a processing enzyme (*e.g*. ubiquitin-specific processing protease) to cleave the ubiquitin from the foreign protein. Through this method, therefore, native foreign protein can be isolated (*e.g*. WO88/024066).

Alternatively, foreign proteins can also be secreted from the cell into the growth media by creating chimeric DNA molecules that encode a fusion protein comprised of a leader sequence fragment that provide for secretion in yeast of the foreign protein. Preferably, there are processing sites encoded between the leader fragment and the foreign gene that can be cleaved either *in vivo* or *in vitro.* The leader sequence fragment usually encodes a signal peptide comprised of hydrophobic amino acids which direct the secretion of the protein from the cell.

DNA encoding suitable signal sequences can be derived from genes for secreted yeast proteins, such as the genes for invertase (EP-A-0012873; JPO 62,096,086) and A-factor (US patent 4,588,684). Alternatively, leaders of non-yeast origin exit, such as an interferon leader, that also provide for secretion in yeast (EP-A-0060057).

A preferred class of secretion leaders are those that employ a fragment of the yeast alpha-factor gene, which contains both a "pre" signal sequence, and a "pro" region. The types of alpha-factor fragments that can be employed include the full-length pre-pro alpha factor leader (about 83 amino acid residues) as well as truncated alpha-factor leaders (usually about 25 to about 50 amino acid residues) (US Patents 4,546,083 and 4,870,008; EP-A-0 324 274). Additional leaders employing an alpha-factor leader fragment that provides for secretion include hybrid alpha-factor leaders made with a presequence of a first yeast, but a pro-region from a second yeast alphafactor. (*e.g*. see WO 89/02463.)

Usually, transcription termination sequences recognized by yeast are regulatory regions located 3' to the translation stop codon, and thus together with the promoter flank the coding sequence. These sequences direct the transcription of an mRNA which can be translated into the polypeptide encoded by the DNA. Examples of transcription terminator sequence and other yeast-recognized termination sequences, such as those coding for glycolytic enzymes.

Usually, the above described components, comprising a promoter, leader (if desired), coding sequence of interest, and transcription termination sequence, are put together into expression constructs. Expression constructs are often maintained in a replicon, such as an extrachromosomal element *(e.g.* plasmids) capable of stable maintenance in a host, such as yeast or bacteria. The replicon may have two replication systems, thus allowing it to be maintained, for example, in yeast for expression and in a prokaryotic host for cloning and amplification. Examples of such yeast-bacteria shuttle vectors include YEp24 [Botstein et al. (1979) Gene 8:17-24], pCl/l [Brake et al. (1984) Proc. Natl. Acad. Sci USA 81:4642-4646], and YRp17 [Stinchcomb et al. (1982) J. Mod. Biol. 158:157]. In addition, a replicon may be either a high or low copy number plasmid. A high copy number plasmid will generally have a copy number ranging from about 5 to about 200, and usually about 10 to about 150. A host containing a high copy number plasmid will preferably have at least about 10, and more preferably at least about 20. Enter a high or low copy number vector may be selected, depending upon the effect of the vector and the foreign protein on the host. See *e.g.* Brake *et al., supra.*

Alternatively, the expression constructs can be integrated into the yeast genome with an integrating vector.

Integrating vectors usually contain at least one sequence homologous to a yeast chromosome that allows the vector to integrate, and preferably contain two homologous sequences flanking the expression construct.

Integrations appear to result from recombinations between homologous DNA in the vector and the yeast chromosome [Orr-Weaver et al. (1983) Methods in Enzymol. 101:228-245]. An integrating vector may be directed to a specific locus in yeast by selecting the appropriate homologous sequence for inclusion in the vector.

See Orr-Weaver *et al., supra.* One or more expression construct may integrate, possibly affecting levels of recombinant protein produced [Rine et al. (1983) Proc. Natl. Acad. Sci. USA 80:6750]. The chromosomal sequences included in the vector can occur either as a single segment in the vector, which results in the integration of the entire vector, or two segments homologous to adjacent segments in the chromosome and flanking the expression construct in the vector, which can result in the stable integration of only the expression construct.

Usually, extrachromosomal and integrating expression constructs may contain selectable markers to allow for the selection of yeast strains that have been transformed. Selectable markers may include biosynthetic genes that can be expressed in the yeast host, such as *ADE2, HIS4, LE U2, TRP1,* and *ALG7,* and the G418 resistance gene, which confer resistance in yeast cells to tunicamycin and G418, respectively. In addition, a suitable selectable marker may also provide yeast with the ability to grow in the presence of toxic compounds, such as metal. For example, the presence of *CUP1* allows yeast to grow in the presence of copper ions [Butt et al. (1987) Microbiol, Rev. 51:351].

Alternatively, some of the above described components can be put together into transformation vectors. Transformation vectors are usually comprised of a selectable marker that is either maintained in a replicon or developed into an integrating vector, as described above.

Expression and transformation vectors, either extrachromosomal replicons or integrating vectors, have been developed for transformation into many yeasts. For example, expression vectors have been developed for, *inter alia,* the following yeasts:Candida albicans [Kurtz, et al. (1986) Mol. Cell. Biol. 6:142], Candida maltosa [Kunze, el al. (1985) J. Basic Microbiol. 25:141]. Hansenula polymorpha [Gleeson, et al. (1986) J. Gen. Microbiol. 132:3459; Roggenkamp et al. (1986) Mol. Gen. Genet. 202:302], Kluyveromyces fragilis [Das, el al. (1984) J. Bacterial. 158:1165], Kluyveromyces lactis [De Louvencourt et al. (1983) J. Bacteriol. 154:737; Van den Berg et al. (1990) Bio/Technology 8:135], Pichia guillerimondii [Kunze et al. (1985) J. Basic Microbiol. 25:141], Pichia pastoris [Cregg, et al. (1985) Mol. Cell. Biol. 5:3376; US Patent Nos. 4,837,148 and 4,929,555], Saccharomyces cerevisiae [Hinnen et al. (1978) Proc. Natl. Acad. Sci. USA 75:1929; Ito et al. (1983) J. Bacteriol. 153:163], Schizosaccharomyces pombe [Beach and Nurse (1981) Nature 300:706], and Yarrowia lipolytica [Davidow, et al. (1985) Curr. Genet. 10:380471 Gaillardin, et al. (1985) Curr. Genet. 10:49].

Methods of introducing exogenous DNA into yeast hosts are well-known in the art, and usually include either the transformation of spheroplasts or of intact yeast cells treated with alkali cations. Transformation procedures usually vary with the yeast species to be transformed. See *e.g*. [Kurtz et al. (1986) Mol. Cell. Biol. 6:142; Kunze et al. (1985) J. Basic Microbiol. 25:141; Candida]; [Gleeson et al. (1986) J. Gen. Microbiol. 132:3459; Roggenkamp et al. (1986) Mol. Gen. Genet. 202:302; Hansenula]; [Das et al. (1984) J. Bacteriol. 158: 1165; De Louvencourt et al. (1983) J. Bacteriol. 154:1165; Van den Berg et al. (1990) Bio/Technology 8:135; Kluyveromyces]; [Cregg et al. (1985) Mol. Cell. Biol. 5:3376; Kunze et al. (1985) J. Basic Microbiol. 25:141; US Patents 4,837,148 & 4,929,555; Pichia]; [Hinnen et al. (1978) Proc. Natl. Acad Sci. USA 75;1929; Ito et al. (1983) J. Bacteriol. 153:163 Saccharomyces]; [Beach & Nurse (1981) Nature 300:706; Schizosaccharomyces]; [Davidow et al. (1985) Curr. Genet. 10:39; Gaillardin et al. (1985) Curr. Genet. 10:49; Yarrowia].

### Pharmaceutical Compositions

Pharmaceutical compositions can comprise polypeptides of the invention. The pharmaceutical compositions will comprise a therapeutically effective amount of polypeptides, of the claimed invention.

The term "therapeutically effective amount" as used herein refers to an amount of a therapeutic agent to treat, ameliorate, or prevent a desired disease or condition, or to exhibit a detectable therapeutic or preventative effect. The effect can be detected by, for example, chemical markers or antigen levels. Therapeutic effects also include reduction in physical symptoms, such as decreased body temperature. The precise effective amount for a subject will depend upon the subject's size and health, the nature and extent of the condition, and the therapeutics or combination of therapeutics selected for administration. Thus, it is not useful to specify an exact effective amount in advance. However, the effective amount for a given situation can be determined by routine experimentation and is within the judgement of the clinician.

A pharmaceutical composition can also contain a pharmaceutically acceptable carrier. The term "pharmaceutically acceptable carrier" refers to a carrier for administration of a therapeutic agent, such as antibodies or a polypeptide, genes, and other therapeutic agents. The term refers to any pharmaceutical carrier that does not itself induce the production of antibodies harmful to the individual receiving the composition, and which may be administered without undue toxicity. Suitable carriers may be large, slowly metabolized macromolecules such as proteins, polysaccharides, polylactic acids, polyglycolic acids, polymeric amino acids, amino acid copolymers, and inactive virus particles. Such carriers are well known to those of ordinary skill in the art.

Pharmaceutically acceptable salts can be used therein, for example, mineral acid salts such as hydrochlorides, hydrobromides, phosphates, sulfates, and the like; and the salts of organic acids such as acetates, propionates, malonates, benzoates, and the like. A thorough discussion of pharmaceutically acceptable excipients is available in Remington's Pharmaceutical Sciences (Mack Pub. Co., N.J. 1991).

Pharmaceutically acceptable carriers in therapeutic compositions may contain liquids such as water, saline, glycerol and ethanol. Additionally, auxiliary substances, such as wetting or emulsifying agents, pH buffering substances, and the like, may be present in such vehicles. Typically, the therapeutic compositions are prepared as injectables, either as liquid solutions or suspensions; solid forms suitable for solution in, or suspension in, liquid vehicles prior to injection may also be prepared. Liposomes are included within the definition of a pharmaceutically acceptable carrier.

### Delivery Methods

Once formulated, compositions can be administered directly to the subject. The subjects to be treated can be animals; in particular, human subjects can be treated.

Direct delivery of the compositions will generally be accomplished by injection, either subcutaneously, intraperitoneally, intravenously or intramuscularly or delivered to the interstitial space of a tissue. The compositions can also be administered into a lesion. Other modes of administration include oral and pulmonary administration, suppositories, and transdermal or transcutaneous applications (*e.g*. see WO98/20734), needles, or hyposprays. Dosage treatment may be a single dose schedule or a multiple dose schedule.

### Vaccines

Vaccines may either be prophylactic (*ie.* to prevent infection) or therapeutic (*ie.* to treat disease after infection).

Such vaccines comprise immunising protein(s), usually in combination with "pharmaceutically acceptable carriers," which include any carrier that does not itself induce the production of antibodies harmful to the individual receiving the composition. Suitable carriers are typically large, slowly metabolized macromolecules such as proteins, polysaccharides, polylactic acids, polyglycolic acids, polymeric amino acids, amino acid copolymers, lipid aggregates (such as oil droplets or liposomes), and inactive virus particles. Such carriers are well known to those of ordinary skill in the art. Additionally, these carriers may function as immunostimulating agents ("adjuvants"). Furthermore, the antigen or immunogen may be conjugated to a bacterial toxoid, such as a toxoid from diphtheria, tetanus, cholera, H. *pylori, etc.* pathogens.

Preferred adjuvants to enhance effectiveness of the composition include, but are not limited to: (1) aluminum salts (alum), such as aluminum hydroxide, aluminum phosphate, aluminum sulfate, etc; (2) oil-in-water emulsion formulations (with or without other specific immunostimulating agents such as muramyl peptides (see below) or bacterial cell wall components), such as for example (a) MF59™ (WO 90/14837; Chapter 10 in Vaccine design: the subunit and adjutant approach, eds. Powell & Newman, Plenum Press 1995), containing 5% Squalene, 0.5% Tween 80, and 0.5% Span 85 (optionally containing various amounts of MTP-PE (see below), although not required) formulated into submicron particles using a microfluidizer such as Model 110Y microfluidizer (Microfluidics, Newton, MA), (b) SAF, containing 10% Squalane, 0.4% Tween 80, 5% pluronic-blocked polymer L121, and thr-MDP (see below) either microfluidized into a submicron emulsion or vortexed to generate a larger particle size emulsion, and (c) Ribi™ adjuvant system (RAS), (Ribi immunochem, Hamilton, MT) containing 2% Squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox^{™}); (3) saponin adjuvants, such as Stimulon^{™} (Cambridge Bioscience, Worcester, MA) may be used or particles generated therefrom such as ISCOMs (immunostimulating complexes); (4) Complete Freund's Adjuvant (CFA) and Incomplete Freund's Adjuvant (IFA); (5) cytokines, such as interleukins *(e.g.* IL-I, IL-2, IL-4, IL-5, lL-6, IL-7, IL-12, *etc*.), interferons *(e.g.* gamma interferon), macrophage colony stimulating factor (M-CSF), tumor necrosis factor (TNF), etc; and (6) other substances that act as immunostimulating agents to enhance the effectiveness of the composition. Alum and MF59™ are preferred.

As mentioned above, muramyl peptides include, but are not limited to, N-acetyl-muramyl-L-threonyl-D-isoglutamine (thr-MDP), N-acetyl-normuramyl-L-alanyl-D-isoglutamine (nor-MDP), N-acetylmuramyl-L-alanyl-D-isoglutaminyl-L-alanine-2-(1'-2'-dipalmitoyl-*sn*-glycero-3-hydroxyphosphoryloxy)-ethylamine (MTP-PE), *etc.*

The immunogenic compositions (*e.g*. the immunising protein, pharmaceutically acceptable carrier, and adjuvant) typically will contain diluents, such as water, saline, glycerol, ethanol, etc. Additionally, auxiliary substances, such as wetting or emulsifying agents, pH buffering substances, and the like, may be present in such vehicles.

Typically, the immunogenic compositions are prepared as injectables, either as liquid solutions or suspensions; solid forms suitable for solution in, or suspension in, liquid vehicles prior to injection may also be prepared. The preparation also may be emulsified or encapsulated in liposomes for enhanced adjuvant effect, as discussed above under pharmaceutically acceptable carriers.

Immunogenic compositions used as vaccines comprise an immunologically effective amount of the antigenic or immunogenic polypeptides, as well as any other of the above-mentioned components, as needed. By "immunologically effective amount", it is meant that the administration of that amount to an individual, either in a single dose or as part of a series, is effective for treatment or prevention. This amount varies depending upon the health and physical condition of the individual to be treated, the taxonomic group of individual to be treated (*e.g*. nonhuman primate, primate, *etc.),* the capacity of the individual's immune system to synthesize antibodies, the degree of protection desired, the formulation of the vaccine, the treating doctor's assessment of the medical situation, and other relevant factors. It is expected that the amount will fall in a relatively broad range that can be determined through routine trials.

The immunogenic compositions are conventionally administered parenterally, *e.g*. by injection, either subcutaneously, intramuscularly, or transdermally/transcutaneously (*e.g*. WO98/20734). Additional formulations suitable for other modes of administration include oral and pulmonary formulations, suppositories, and transdermal applications. Dosage treatment may be a single dose schedule or a multiple dose schedule. The vaccine may be administered in conjunction with other immunoregulatory agents.

### BRIEF DESCRIPTION OF THE DRAWINGS

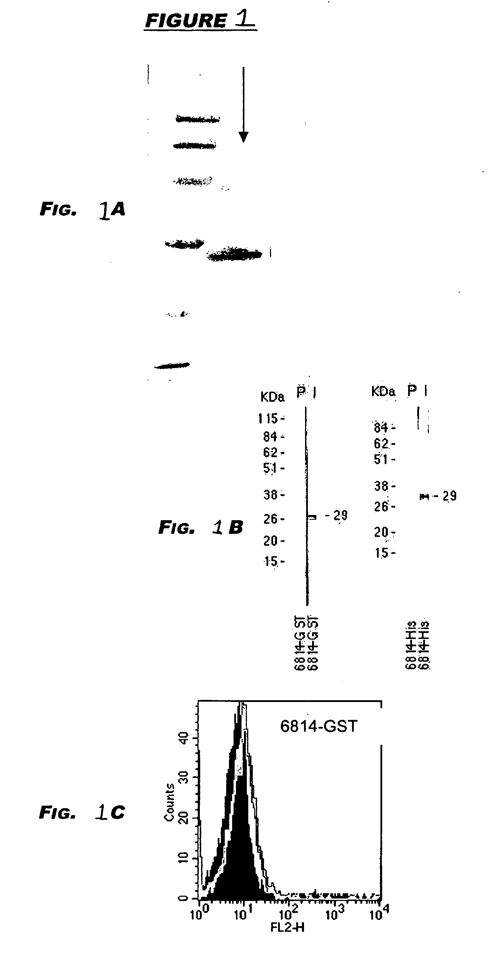

**Figure 1** shows data pertaining to example 1.

### EXAMPLE

The example indicates a *C.pneumoniae* protein, together with evidence to support the view that the protein is a useful antigen for vaccine production and development or for diagnostic purposes. This evidence takes the form of:
- Computer prediction based on sequence information from CWL029 strain (*e.g*. using the PSORT algorithm available from *www.psort.nibb.ac₋jp).*
- Data on recombinant expression and purification of the proteins cloned from IOL207 strain.
- Western blots to demonstrate immunoreactivity in serum (typically a blot of an EB extract of *C.pneumoniae* strain FB/96 stained with mouse antiserum against the recombinant protein).
- FACTS analysis of *C.pneumoniae* bacteria or purified EBs to confirm accessibility of the antigen to the immune system (see also table III).

Various tests can be used to assess the *in vivo* immunogenicity of the proteins identified in the example. For example, the protein can be expressed recombinantly and used to screen patient sera by immunoblot. A positive reaction between the protein and patient serum indicates that the patient has previously mounted an immune response to the protein in question *ie.* the protein is an immunogen. This method can also be used to identify immunodominant proteins.

The recombinant protein can also be conveniently used to prepare antibodies e.g. in a mouse. These can be used for direct confirmation that a protein is located on the cell-surface. Labelled antibody (e.g. fluorescent labelling for FACS) can be incubated with intact bacteria and the presence of label on the bacterial surface confirms the location of the protein.

In particular, the following methods (A) to (O) were used to express, purify and biochemically characterise the proteins of the invention:

### CLONING OF CPN ORFs FOR EXPRESSION IN E.COLI

ORFs of *Chlamydia pneumoniae* (Cpn) were cloned in such a way as to potentially obtain three different kind of proteins:
a) proteins having an hexa-histidine tag at the C-terminus (cpn-His)
b) proteins having a GST fusion partner at the N-terminus (Gst-cpn)
c) proteins having both hexa-histidine tag at the C-terminus and GST at the N-terminus (GST/His fusion; NH₂-GST-cpn-(His)₆-COOH)

The type a) proteins were obtained upon cloning in the pET21b+ (Novagen). The type b) and c) proteins were obtained upon cloning in modified pGEX-KG vectors [Guan & Dixon (1991) Anal. Biochem. 192:262]. For instance pGEX-KG was modified to obtain pGEX-NN, then by modifying pGEX-NN to obtain pGEX-NNH. The Gst-cpn and Gst-cpn-His proteins were obtained in pGEX-NN and pGEX-NNH respectively.

The modified versions of pGEX-KG vector were made with the aim of allowing the cloning of single amplification products in all three vectors after only one double restriction enzyme digestion and to minimise the presence of extraneous amino acids in the final recombinant proteins.

### (A) Construction of pGEX-NN and pGEX-NNH expression vectors

Two couples of complementary oligodeoxyribonucleotides were synthesised using the DNA synthesiser ABI394 (Perkin Elmer) and the reagents from Cruachem (Glasgow, Scotland). Equimolar amounts of the oligo pairs (50 ng each oligo) were annealed in T4 DNA ligase buffer (New England Biolabs) for 10 min in a final volume of 50µl and then were left to cool slowly at room temperature.

With the described procedure he following DNA linkers were obtained:

gexNN linker:

NdeI  NheI  XmaI  EcoRI  NcoI  SalI  XhoI  SacI  NotI
GATCCCATATGGCTAGCCCGGGGAATTCGTCCATGGAGTGAGTCGACTGACTCGAGTGATCGAGCTCCTGAGCGGCCGCATGAA
GGTATACCGATCGGGCCCCTTAAGCAGGTACCTCACTCAGCTGACTGAGCTCACTAGCTCGAGGACTCGCCGGCGTACTTTCGA

gexNNH linker:
HindIII NotI XhoI --Hexa-Histidine--
TCGACAAGCTTGCGGCCGCACTCGAGCATCACCATCACCATCACTGAT
GTTCGAACGCCGGCGTGAGCACGTAGAGGTAGTGGTAGTGACTATCGA

The plasmid pGEX-KG was digested with BamHI and HindIII and 100 ng were ligated overnight at 16°C to the linker gexNN with a molar ratio of 3:1 linker/plasmid using 200 units of T4 DNA ligase (New england Biolabs). After transformation of the ligation product in *E. coli* DH5, a clone containing the pGEX-NN plasmid, having the correct linker, was selected by means of restriction enzyme analysis and DNA sequencing.

The new plasmid pGEX-NN was digested with SalI and HindIII and ligated to the linker gexNNH. After transformation of the ligation product in *E*. *coli* DH5, a clone containing the pGEX-NNH plasmid, having the correct linker, was selected by means of restriction enzyme analysis and DNA sequencing.

### (B) Chromosomal DNA preparation

The chromosomal DNA of elementary bodies (EB) of *C.pneumoniae* strain 10L-207 was prepared by adding 1.5ml of lysis buffer (10 mM Tris-HCl, 150 mM NaCl, 2 mM EDTA, 0,6 % SDS, 100 µg/ml Proteinase K, pH 8) to 450 µl EB suspension (400.000/µl) and incubating overnight at 37°C. After sequential extraction with phenol, phenol-chloroform, and chloroform, the DNA was precipitated with 0,3 M sodium acetate, pH 5,2 and 2 volumes of absolute ethanol. The DNA pellet was washed with 70 % ethanol. After solubilization with distilled water and treatment with 20 µg/ml RNAse A for 1 hour at RT, the DNA was extracted again with phenol-chloroform, alcohol precipitated and suspended with 300 µl 1 mM Tris-HCl pH 8,5. The DNA concentration was evaluated by measuring OD₂₆₀ of the sample.

### (C) Oligonucleotide design

Synthetic oligonucleotide primers were designed on the basis of the coding sequence of each ORF using the sequence of *C.pneumoniae* strain CWL029. Any predicted signal peptide were omitted, by deducing the 5' end amplification primer sequence immediately downstream from the predicted leader sequence. For most ORFs, the 5' tail of the primers (table I) included only one restriction enzyme recognition site (NdeI, or NheI, or SpeI depending on the gene's own restriction pattern); the 3' primer tails (tableI) included a XhoI or a NotI or a HindIII restriction site.

**Table I. Oligonucleotide tails of the primers used to amplify Cpn genes.**

| 5' tails | | 3' tails | |
|---|---|---|---|
| NdeI | 5' GTGCGT**CATATG** 3' | XhoI | 5' GCG**TCTCGAG** 3' |
| NheI | 5' GTGCGT**GCTAGC** 3' | NotI | 5' ACTCGCTA**GCGGCCGC** 3' |
| SpeI | 5' GTGCGT**ACTAGT3**' | HindIII | 5' GCGT**AAGCTT** 3' |

As well as containing the restriction enzyme recognition sequences, the primers included nucleotides which hybridized to the sequence to be amplified. The number of hybridizing nucleotides depended on the melting temperature of the primers which was determined as described [(Breslauer et al. (1986) PNAS USA 83:3746-50]. The average melting temperature of the selected oligos was 50-55°C for the hybridizing region alone and 65-75°C for the whole oligos. Table II shows the forward and reverse primers used for each amplification.

### (D) Amplification

The standard PCR protocol was as follow: 50 ng genomic DNA were used as template in the presence of 0,2 µM each primer, 200 µM each dNTP, 1,5 mM MgCl₂, 1x PCR buffer minus Mg (Gibco-BRL), and 2 units of Taq DNA polymerase (Platinum Taq, Gibco-BRL) in a final volume of 100 µl. Each sample underwent a double-step amplification: the first 5 cycles were performed using as the hybridizing temperature the one of the oligos excluding the restriction enzyme tail, followed by 25 cycles performed according to the hybridization temperature of the whole lenght primers. The standard cycles were as follow:

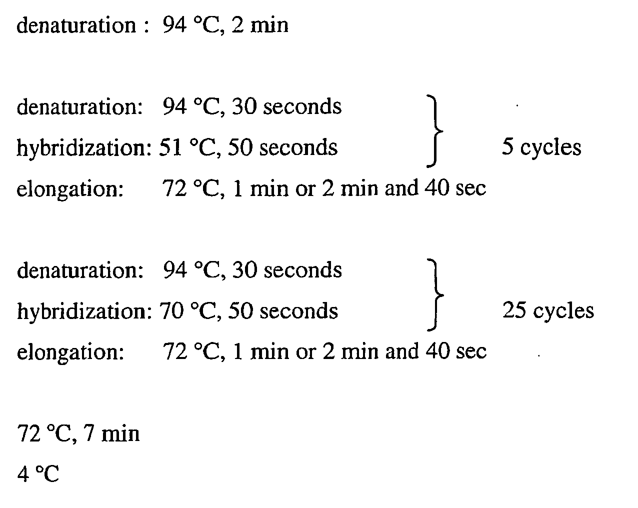

The elongation time was 1 min for ORFs shorter than 2000 bp, and 2 min and 40 seconds for ORFs longer than 2000 bp. The amplifications were performed using a Gene Amp PCR system 9600 (Perkin Elmer).

To check the amplification results, 4 µl of each PCR product was loaded onto 1-1.5 agarose gel and the size of amplified fragments compared with DNA molecular weight standards (DNA markers III or IX, Roche). The PCR products were loaded on agarose gel and after electrophoresis the right size bands were excised from the gel. The DNA was purified from the agarose using the Gel Extraction Kit (Qiagen) following the instruction of the manufacturer. The final elution volume of the DNA was 50 µl TE (10 mM Tris-HCl, 1 mM EDTA, pH 8). One µl of each purified DNA was loaded onto agarose gel to evaluate the yield.

### (E) Digestion of PCR fragments

One-two µg of purified PCR product were double digested overnight at 37 °C with the appropriate restriction enzymes (60 units of each enzyme) using the appropriate restriction buffer in 100 µl final volume. The restriction enzymes and the digestion buffers were from New England Biolabs. After purification of the digested DNA (PCR purification Kit, Qiagen) and elution with 30 µl TE, 1 µl was subjected to agarose gel electrophoresis to evaluate the yield in comparison to titrated molecular weight standards (DNA markers III or IX, Roche).

### (F) Digestion of the cloning vectors (pET21b+, pGEX-NN, and pGEX-NNH)

10 µg of plasmid was double digested with 100 units of each restriction enzyme in 400 µl reaction volume in the presence of appropriate buffer by overnight incubation at 37 °C. After electrophoresis on a 1% agarose gel, the band corresponding to the digested vector was purified from the gel using the Qiagen Qiaex II Gel Extraction Kit and the DNA was eluted with 50 µl TE. The DNA concentration was evaluated by measuring OD₂₆₀ of the sample.

### (G) Cloning

75ng of the appropriately digested and purified vectors and the digested and purified fragments corresponding to each ORF, were ligated in final volumes of 10-20 µl with a molar ratio of 1:1 fragment/vector, using 400 units T4 DNA ligase (New England Biolabs) in the presence of the buffer supplied by the manufacturer. The reactions were incubated overnight at 16 °C.

Transformation in *E coli* DH5 competent cells was performed as follow: the ligation reaction was mixed with 200 µl of competent DH5 cells and incubated on ice for 30 min and then at 42 °C for 90 seconds. After cooling on ice, 0.8 ml LB was added and the cells were incubated for 45 min at 37 °C under shaking. 100 and 900 µl of cell suspensions were plated on separate plates of agar LB 100 µg/ml Ampicillin and the plates were incubated overnight at 37°C. The screening of the transformants was done by growing randomly chosen clones in 6 ml LB 100 µg/ml Ampicillin, by extracting the DNA using the Qiagen Qiaprep Spin Miniprep Kit following the manufacturer instructions, and by digesting 2 µl of plasmid minipreparation with the restriction enzymes specific for the restriction cloning sites. After agarose gel electrophoresis of the digested plasmid mini-preparations, positive clones were chosen on the basis of the correct size of the restriction fragments, as evaluated by comparison with appropriate molecular weight markers (DNA markers III or IX, Roche).

### (H) Expression

1 µl of each right plasmid mini-preparation was transformed in 200 µl of competent *E*. *coli* strain suitable for expression of the recombinant protein. All pET21b+ recombinant plasmids were transformed in BL21 DE3 (Novagen) *E*. *coli* cells, whilst all pGEX-NN and all pGEX-NNH recombinant plasmids were transformed in BL21 cells (Novagen). After plating transformation mixtures on LB/Amp agar plates and incubation overnight at 37 °C, single colonies were inoculated in 3 ml LB 100 µg/ml Ampicillin and grown at 37 °C overnight. 70 µl of the overnight culture was inoculated in 2 ml LB/Amp and grown at 37 °C until OD₆₀₀ of the pET clones reached the 0,4-0,8 value or until OD₆₀₀ of the pGEX clones reached the 0,8-1 value. Protein expression was then induced by adding IPTG (Isopropil β-D thio-galacto-piranoside) to the mini-cultures. pET clones were induced using 1 mM IPTG, whilst pGEX clones were induced using 0.2 mM IPTG. After 3 hours incubation at 37 °C the final OD₆₀₀ was checked and the cultures were cooled on ice. After centrifugation of 0.5 ml culture, the cell pellet was suspended in 50 µl of protein Loading Sample Buffer (60 mM TRIS-HCl pH 6.8, 5% w/v SDS, 10% v/v glycerin, 0.1% w/v Bromophenol Blue, 100 mM DTT) and incubated at 100 °C for 5 min. A volume of boiled sample corresponding to 0.1 OD₆₀₀ culture was analysed by SDS-PAGE and Coomassie Blue staining to verify the presence of induced protein band.

### PURIFICATION OF THE RECOMBINANT PROTEINS

Single colonies were inoculated in 25 ml LB 100 µg/ml Ampicillin and grown at 37 °C overnight. The overnight culture was inoculated in 500 ml LB/Amp and grown under shaking at 25 °C until OD₆₀₀ 0,4-0,8 value for the pET clones, or until OD₆₀₀ 0,8-1 value for the pGEX clones. Protein expression was then induced by adding IPTG to the cultures. pET clones were induced using 1 mM IPTG, whilst pGEX clones were induced using 0.2 mM IPTG. After 4 hours incubation at 25 °C the final OD₆₀₀ was checked and the cultures were cooled on ice. After centrifugation at 6000 rpm (JA10 rotor, Beckman), the cell pellet was processed for purification or frozen at -20°C.

### (I) Procedure for the purification of soluble His-tagged proteins from E.coli

1. Transfer the pellets from -20°C to ice bath and reconstitute with 10 ml 50 mM NaHPO₄ buffer, 300 mM NaCl, pH 8,0, pass in 40-50 ml centrifugation tubes and break the cells as per the following outline:
2. Break the pellets in the French Press performing three passages with in-line washing.
3. Centrifuge at about 30-40000 x g per 15-20 min. If possible use rotor JA 25.50 (21000 rpm, 15 min.) or JA-20 (18000 rpm, 15 min.)
4. Equilibrate the Poly-Prep columns with 1 ml Fast Flow Chelating Sepharose resin with 50 mM phosphate buffer, 300 mM NaCl, pH 8,0.
5. Store the centrifugation pellet at -20°C, and load the supernatant in the columns.
6. Collect the flow through.
7. Wash the columns with 10 ml (2 ml + 2 ml + 4 ml) 50 mM phosphate buffer, 300 mM NaCl, pH 8,0.
8. Wash again with 10 ml 20 mM imidazole buffer, 50 mM phosphate, 300 mM NaCl, pH 8,0.
9. Elute the proteins bound to the columns with 4,5 ml (1,5 ml + 1,5 ml + 1,5 ml) 250 mM imidazole buffer, 50 mM phosphate, 300 mM NaCl, pH 8,0 and collect the 3 corresponding fractions of ∼ 1,5 ml each. Add to each tube 15 µl DTT 200 mM (final concentration 2 mM)
10. Measure the protein concentration of the first two fractions with the Bradford method, collect a 10 µg aliquot of proteins from each sample and analyse by SDS-PAGE. (N.B.: should the sample be too diluted, load 21 µl + 7 µl loading buffer).
11. Store the collected fractions at +4°C while waiting for the results of the SDS-PAGE analysis.
12. For immunisation prepare 4-5 aliquots of 100 µg each in 0,5 ml in 40% glycerol. The dilution buffer is the above elution buffer, plus 2 mM DTT. Store the aliquots at -20°C until immunisation.

### (J) Purification of His-tagged proteins from Inclusion bodies

Purifications were carried out essentially according the following protocol:
1. Bacteria are collected from 500 ml cultures by centrifugation. If required store bacterial pellets at -20°C. For extraction, resuspend each bacterial pellet in 10 ml 50 mM TRIS-HCl buffer, pH 8,5 on an ice bath.
2. Disrupt the resuspended bacteria with a French Press, performing two passages.
3. Centrifuge at 35000 x g for 15 min and collect the pellets. Use a Beckman rotor JA 25.50 (21000 rpm, 15 min.) or JA-20 (18000 rpm, 15 min.).
4. Dissolve the centrifugation pellets with 50 mM TRIS-HCl, 1 mM TCEP {Tris(2-carboxyethyl)-phosphine hydrochloride, Pierce} , 6M guanidium chloride, pH 8,5. Stir for ∼ 10 min. with a magnetic bar.
5. Centrifuge as described above, and collect the supernatant..
6. Prepare an adequate number of Poly-Prep (Bio-Rad) columns containing 1 ml of Fast Flow Chelating Sepharose (Pharmacia) saturated with Nichel according to manufacturer recommendations.. Wash the columns twice with 5 ml of H₂O and equilibrate with 50 mM TRIS-HCl, 1 mM TCEP, 6M guanidinium chloride, pH 8,5.
7. Load the supernatants from step 5 onto the columns, and wash with 5 ml of 50 mM TRIS-Hcl buffer, 1 mM TCEP, 6M urea, pH 8,5
8. Wash the columns with 10 ml of 20 mM imidazole, 50 mM TRIS-HCl , 6M urea, 1 mM TCEP, pH 8,5. Collect and set aside the first 5 ml for possible further controls.
9. Elute the proteins bound to the columns with 4,5 ml of a buffer containing 250 mM imidazole, 50 mM TRIS-HCl, 6M urea, 1 mM TCEP, pH 8,5. Add the elution buffer in three 1,5 ml aliquots, and collect the corresponding 3 fractions. Add to each fraction 15 µl DTT (final concentration 2 mM) .
10. Measure eluted protein concentration with the Bradford method, and analyze aliquots of ca 10 µg of protein by SDS-PAGE.
11. Store proteins at -20°C in 40% (v/v) glycerol, 50 mM TRIS-HCl, 2M urea, 0.5 M arginine, 2 mM DTT, 0.3 mM TCEP, 83.3 mM imidazole, pH 8,5

### (K) Procedure for the purification of GST-fusion proteins from E.coli

1. Transfer the bacterial pellets from -20°C to an ice bath and resuspend with 7,5 ml PBS, pH 7,4 to which a mixture of protease inhibitors (C∅MPLETE™ - Boehringer Mannheim, 1 tablet every 25 ml of buffer) has been added. Transfer to 40-50 ml centrifugation tubes and sonicate according to the following procedure:
   a) Position the probe at about 0,5 cm from the bottom of the tube
   b) Block the tube with the clamp
   c) Dip the tube in an ice bath
   d) Set the sonicator as follows: Timer → Hold, Duty Cycle → 55, Out. Control → 6.
   e) perform 5 cycles of 10 impulses at a time lapse of 1 minute (i.e. one cycle = 10 impulses + ∼45" hold; b. 10 impulses + ∼45" hold; c. 10 impulses + ∼45" hold; d. 10 impulses + ∼45" hold; e. 10 impulses + ∼45" hold)
2. Centrifuge at about 30-40000 x g for 15-20 min. E.g.: use rotor Beckman JA 25.50 at 21000 rpm, for 15 min.
3. Store the centrifugation pellets at -20°C, and load the supernatants on the chromatography columns, as follows
4. Equilibrate the Poly-Prep (Bio-Rad) columns with 0,5 ml (≅1 ml suspension) of Glutathione-Sepharose 4B resin, wash with 2 ml (1+1) H₂O, and then with 10 ml (2 + 4 + 4) PBS, pH 7,4.
5. Load the supernatants on the columns and discard the flow through.
6. Wash the columns with 10 ml (2 + 4 + 4) PBS, pH 7,4.
7. Elute the proteins bound to the columns with 4,5 ml of 50 mM TRIS buffer, 10 mM reduced glutathione, pH 8.0, adding 1,5 ml + 1,5 ml + 1,5 ml and collecting the respective 3 fractions of ∼1,5 ml each.
8. Measure the protein concentration of the first two fractions with the Bradford method, analyse a 10 µg aliquot of proteins from each sample by SDS-PAGE. (N.B.: if the sample is too diluted load 21 µl (+ 7 µl loading buffer).
9. Store the collected fractions at +4°C while waiting for the results of the SDS-PAGE analysis.
10. For each protein destined to the immunisation prepare 4-5 aliquots of 100 µg each in 0,5 ml of 40% glycerol. The dilution buffer is 50 mM TRIS.HCl, 2 mM DTT, pH 8,0. Store the aliquots at -20°C until immunisation..

### SEROLOGY

### (L) Protocol of immunization

1. Groups of four CD1 female mice aged between 6 and 7 weeks were immunized with 20 µg of recombinant protein resuspended in 100 µl.
2. Four mice for each group received 3 doses with a 14 days interval schedule.
3. Immunization was performed through intra-peritoneal injection of the protein with an equal volume of Complete Freund's Adjuvant (CFA) for the first dose and Incomplete Freund's Adjuvant (IFA) for the following two doses.
4. Sera were collected before each immunization. Mice were sacrified 14 days after the third immunization and the collected sera were pooled and stored at -20°C.

### (M) Western blot analysis of Cpn elementary body proteins with mouse sera

Aliquots of elementary bodies containing approximately 4 µg of proteins, mixed with SDS loading buffer (1x: 60 mM TRIS-HCl pH 6.8, 5% w/v SDS, 10% v/v glycerin, 0.1% Bromophenol Blue, 100 mM DTT) and boiled 5 minutes at 95° C, were loaded on a 12% SDS-PAGE gel. The gel was run using a SDS-PAGE running buffer containing 250 mM TRIS, 2.5 mM Glycine and 0.1 %SDS. The gel was electroblotted onto nitrocellulose membrane at 200 mA for 30 minutes. The membrane was blocked for 30 minutes with PBS, 3% skimmed milk powder and incubated O/N at 4° C with the appropriate dilution (1/100) of the sera. After washing twice with PBS + 0.1% Tween (Sigma) the membrane was incubated for 2 hours with peroxidase-conjugated secondary anti-mouse antibody (Sigma) diluted 1:3000. The nitrocellulose was washed twice for 10 minutes with PBS + 0.1% Tween-20 and once with PBS and thereafter developed by Opti-4CN Substrate Kit (Biorad).

Lanes shown in Western blots are: (P) = pre-immune control serum; (I) = immune serum.

### (N) FACS analysis of Chlamydia pneumoniae elementary bodies with mouse sera

1. 2x10⁵ Elementary Bodies (EB)/well were washed with 200 µl of PBS-0.1%BSA in a 96 wells U bottom plate and centrifuged for 10 min. at 1200rpm, at 4°C.
2. The supernatant was discarded and the E.B. resuspended in 10 µl of PBS-0.1%BSA.
3. 10µl mouse sera diluted in PBS-0.1%BSA were added to the E.B. suspention to a final dilution of 1:400, and incubated on ice for 30 min.
4. EB were washed by adding 180µl PBS-0.1%BSA and centrifuged for 10min. at 1200rpm, 4°C.
5. The supernatant was discarded and the E.B. resuspended in 101 of PBS-0.1%BSA.
6. 10µl of a goat anti-mouse IgG, F(ab')₂ fragment specific-R-Phycoerythrin-conjugated (Jackson Immunoresearch Laboratories Inc., cat.N°115-116-072) was added to the EB suspension to a final dilution of 1:100, and incubated on ice for 30 min. in the dark.
7. EB were washed by adding 180µl PBS-0.1%BSA and centrifuged for 10min. at 1200rpm, 4°C.
8. The supernatant was discarded and the E.B. resuspended in 150 µl of PBS-0.1%BSA.
9. E.B. suspension was passed through a cytometric chamber of a FACS Calibur (Becton Dikinson, Mountain View, CA USA) and 10.000 events were acquired.
10. Data were analysed using Cell Quest Software (Becton Dikinson, Mountain View, CA USA) by drawing a morphological dot plot (using forward and side scatter parameters) on E.B. signals. An histogram plot was then created on FL2 intensity of fluorescence log scale recalling the morphological region of EB.

NB: the results of FACS depend not only on the extent of accessibility of the native antigens but also on the quality of the antibodies elicited by the recombinant antigens, which may have structures with a variable degree of correct folding as compared with the native protein structures. Therefore, even if a FACS assay appears negative this does not necessarily mean that the protein is not abundant or accessible on the surface. PorB antigen, for instance, gave negative results in FACS but is a surface-exposed neutralising antigen [Kubo & Stephens (2000) Mol. Microbiol. 38:772-780].

### Example 1

The following *C.pneumoniae* protein (PID 4376814) was expressed <SEQ ID 117; cp6814>:

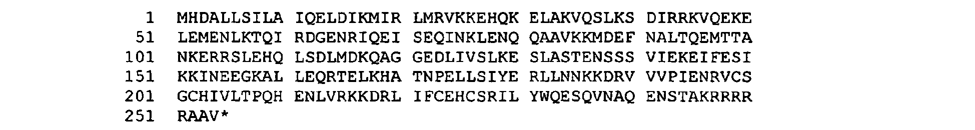

The cp6814 nucleotide sequence <SEQ ID 118> is:

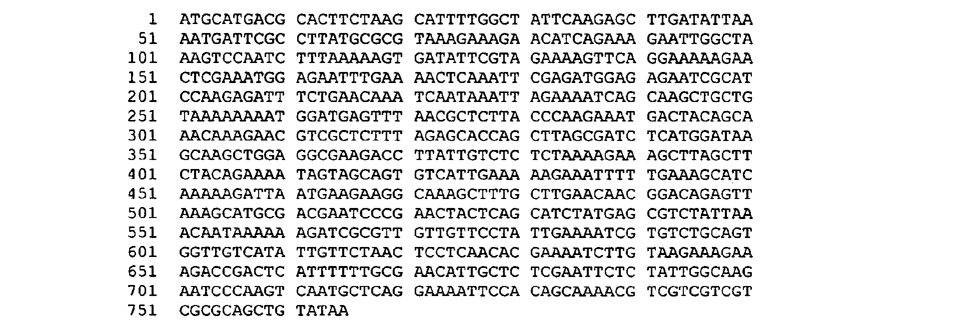

The PSORT algorithm predicts an inner membrane location (0.070).

The protein was expressed in *E.coli* and purified as a GST-fusion (Figure 1A) or his-tagged product. The recombinant proteins were used to immunise mice, whose sera were used in Western blot (Figure 1B) and FACS (Figure 1C) analyses.

These experiments show that cp6814 is a surface-exposed and immunoaccessible protein, and that it is a useful immunogen. These properties are not evident from the sequence alone.

**TABLE II - sequences of the primers used to amplify Cpn genes.**

| **Orf ID** | **N-terminus final primer** | **C-terminus final primer** |
|---|---|---|
| CP6814P | GTGCGT CATATG CATGACGCACTTCTAAG | GCGT CTCGAG TACAGCTGCGCGA |

**TABLE III - results in FACS analysis**

| **cp number** | **Molecular Weight (kDa)** | | **Fusion type** |
|---|---|---|---|
| | **Theoretical** | **Western Blot** | |
| 6814 | 29.6 | 28 | GST |

## Claims

1. A protein comprising amino acid sequence SEQ ID NO: 117 for use in the treatment or prevention of infection due to Chlamydia bacteria.

2. A protein according to claim 1, wherein the Chlamydia bacteria is *C. pneumoniae.*

3. The use of a protein according to claim 1 or claim 2 in the manufacture of a medicament for the treatment or prevention of infection due to *Chlamydia* bacteria, particularly *Chlamydia pneumoniae.*

## Patentansprüche

1. Protein, das die Aminosäuresequenz SEQ ID NO:117 umfaßt, zur Verwendung bei der Behandlung oder Vermeidung einer Infektion aufgrund von Chlamydia-Bakterien.

2. Protein nach Anspruch 1, wobei die Chlamydia-Bakterien *C. pneumoniae* sind.

3. Verwendung eines Proteins nach Anspruch 1 oder Anspruch 2 bei der Herstellung eines Medikaments für die Behandlung oder Vermeidung einer Infektion aufgrund von Chlamydia-Bakterien, insbesondere *Chlamydia pneumoniae.*

## Revendications

1. Protéine comprenant la séquence d'acides aminés SÉQ ID NO : 117 en vue d'une utilisation dans le traitement ou la prévention d'une infection due à la bactérie Chlamydia.

2. Protéine selon la revendication 1, où la bactérie Chlamydia est *C*. *pneumoniae.*

3. Utilisation d'une protéine selon la revendication 1 ou la revendication 2, dans la fabrication d'un médicament destiné au traitement ou à la prévention d'une infection due à la bactérie Chlamydia, en particulier *Chlamydia pneumoniae.*
